# EUROPÄISCHE PATENTSCHRIFT

(11) **EP 1 609 773 B1**
(45) Veröffentlichungstag und Bekanntmachung des Hinweises auf die Patenterteilung: **08.02.2012**
(21) Anmeldenummer: 05450109.3
(22) Anmeldetag: 22.06.2005
(51) Int. Cl.: C05F 17/00, C05F 17/02, C05F 9/04, A61L 11/00

(54) **Verfahren zum Belüften von Rotte sowie Vorrichtung zur Durchführung dieses Verfahrens**
Method of aerating compost and device to carry out the method
Procédé d'aération de compost et appareil pour la mise en oeuvre de ce procédé

(30) Priorität: 22.06.2004 AT 10592004
(43) Veröffentlichungstag der Anmeldung: 28.12.2005
(73) Patentinhaber: Harrer, Ewald, 3544 Idolsberg (AT)
(72) Erfinder: Hanke, Reinhart, 8700 Leoben (AT); Harrer, Ewald, 1040 Wien (AT)
(74) Vertreter: Haffner, Thomas M.

(56) Entgegenhaltungen:
- EP-A- 0 949 223
- CH-A5- 648 011

## Beschreibung

Die Erfindung bezieht sich auf ein Verfahren zum Belüften von Rotte bei welcher die Luft von unten nach oben durch den Rottekörper gedrückt wird, wobei die durch die Rotte drückend hindurchgeleitete Luft alternierend von Luft mit verschiedenen physikalischen und chemischen Eigenschaften und von an geometrisch voneinander verschiedenen Stellen eingeleiteter Luft gebildet wird.

Verfahren zur mechanischen Belüftung von Rotte sind in unterschiedlicher Ausbildung bekannt. Für den ersten Prozessabschnitt des aeroben Rotteprozesses, die sogenannte Intensivrotte, wird gegenwärtig üblicherweise in geschlossenen Reaktoren belüftet, wobei in diesem Prozessabschnitt der größte Teil der im Einsatzmaterial enthaltenen leicht abbaubaren organischen Substanz unter Bildung neuer Biomasse mikrobiell abgebaut wird. Die Belüftung kann hierbei entweder durch Einpressen von Prozessluft von unten nach oben durch den Rottekörper oder durch Absaugen von Luft aus der Bodenschicht des Rottekörpers vorgenommen werden. Im ersten Fall durchströmt die Prozessluft den Rottekörper von unten nach oben, wobei ein geringer Überdruck angewandt wird. Bei der sogenannten saugenden Belüftung durchströmt die Prozessluft den Rottekörper in umgekehrter Richtung, wobei die Prozessabluft aus der Bodenschicht abgesaugt wird. Bei saugender Belüftung muss mit entsprechend großem Unterdruck gearbeitet werden, wobei sich im gesamten Belüftungssystem relativ hohe Druckunterschiede zur Atmosphäre ergeben und die Gefahr besteht, dass im Bereich der Absaugung am Boden des Reaktors Düsenreihen oder Belüftungsrinnen verstopft werden.

Demgegenüber haben Systeme mit drückender Belüftung, bei welchen mit geringem Überdruck gearbeitet wird, konstruktiv und betrieblich eine Reihe von Vorteilen, und hier zuallererst eine verringerte Verstopfungsgefahr der Belüftungselemente und geringere Druckverluste des Rottekörpers durch kontinuierliche Belüftung. Insgesamt treten im Falle von kontinuierlicher Belüftung kleinere Luftgeschwindigkeiten als bei einer intermittierenden Belüftung auf. Die Gefahr einer Kondensatbildung im Bereich der Ablufterfassung kann wesentlich herabgesetzt oder sogar eliminiert werden. Neben der geringeren Druckunterschiede zur Atmosphäre im gesamten Belüftungssystem ergibt sich insgesamt eine vergleichmäßigung der Luftbeaufschlagung des Rottekörpers durch Einsatz von Umluft, d.h. durch Rückführung von Prozessabluft als Prozessrohluft. Alle diese Vorteile sind allerdings auch mit nicht unerheblichen Nachteilen verbunden, wobei hier insbesondere auf die geringe Wirkung der Nachbefeuchtung des Rottegutes verwiesen werden muss, da diese gegen den Luftstrom von oben in das Rottegut eingebracht werden muss. Die mitunter sehr rasche Abtrocknung des Rottegutes von unten nach oben kann zur Ausbildung bevorzugt durchströmter Zonen in vertikaler Richtung führen. Diese sogenannte Kaminbildung als Folge ungleichmäßiger Abtrocknung wird durch stationäre Strömungsbedingungen und Strömungsbilder der Prozessluft begünstigt. Durch vermehrten Einsatz von Umluft zur Verbesserung der Luftverteilung können diese Nachteile in keiner Weise vollständig kompensiert werden, wobei sich zu allem Überfluss dann die gesamten Druckverluste im Bereich des Rottekörpers erhöhen würden.

EP 0949223 offenbart ein Verfahren zum Belüften von Rotte, bei welcher die Luft von unter nach oben der Rottekörper gedrückt wird.

Die vorliegende Erfindung zielt darauf ab, bei einer drückenden Belüftung die Kaminbildung zu verhindern und die Abtrocknungsgeschwindigkeit des Rottekörpers herabzusetzen. Zur Lösung dieser Aufgabe besteht das erfindungsgemäße Verfahren im wesentlichen darin, dass Belüftungselemente in Gruppen unterteilt sind, die Gruppen von Belüftungselementen von Luft mit voneinander verschiedener Zusammensetzung beaufschlagt werden und die Beaufschlagung mittels einer Belüftungsweiche zwischen den Gruppen gewechselt wird. Die alternierende Belüftung des Rottekörpers führt zur Behinderung der Ausbildung stationärer Strömungsbedingungen und stationärer Strömungsbilder, wodurch Kaminbildungen hintangehalten werden. Die Maßnahme, Rohluft mit unterschiedlicher Qualität einzusetzen, bei welcher somit Belüftungselemente abwechselnd mit Rohluft unterschiedlicher Eigenschaften beschickt werden, führt zur Verminderung der Abtrocknungsgeschwindigkeit des Rottekörpers durch Senkung des spezifischen Nettowasserverbrauchs zur Prozesskühlung. Es kann hierbei beispielsweise wechselweise mit Frischluft und Umluft als Rohluft beschickt werden, wobei durch Einsatz von beispielsweise wasserdampfgesättigter Rohluft unmittelbar die entsprechende Feuchte wiederum eingetragen werden kann. Eine wasserdampfgesättigte Rohluft kann durch entsprechende Rohluftkonditionierung der Frischluft bzw. der Umluft erfolgen.

In besonders vorteilhafter Weise wird das erfindungsgemäße Verfahren so durchgeführt, dass Rohluft als Frischluft, Umluft und/oder konditionierte Rohluft mit unterschiedlichem Wasserdampfgehalt, unterschiedlicher Temperatur und/oder unterschiedlicher Konditionierung alternierend durch Belüftungselemente geleitet wird. Insgesamt wird hier bei einem minimalen Regelungsaufwand unterschiedlichen Rottezusammensetzungen Rechnung getragen, wobei die eingangs gesetzten Ziele auch bei unterschiedlichen Ausgangsmaterialien erreicht werden können. Mit Vorteil wird hierbei so vorgegangen, dass die alternierend beschickten Belüftungselemente von mit anderer Rohluft beschickten Belüftungselementen umgeben sind oder diese zwischen sich einschließen.

Das beschriebene Prinzip der alternierenden Belüftung besteht darin, dass beispielsweise mit zwei Rohluftströmen unterschiedlicher Qualität und/oder Menge, z. B. mit vorzugsweise konditionierter Frischluft und Umluft gearbeitet wird, wobei die jeweiligen mit einer bestimmten Luftqualität beschickten Reihen bzw. Gruppen alternierend gewechselt werden können. Luftströme mit unterschiedlicher Temperatur, unterschiedlichem Druck bzw. Menge und/oder unterschiedlichem Wassergehalt haben naturgemäß unterschiedlichen Einfluss auf die Ausbildung von Durchströmungswegen im Rottekörper und insbesondere auf die Kaminbildung, wie sie beispielsweise durch Abtrocknung eintreten kann. Durch alternierende Beaufschlagung der Belüftungselemente können derartige Veränderungen so weit vergleichmäßigt werden, dass eine Kaminbildung nicht auftreten kann. Die jeweils eingesetzte Belüftungsweiche kann nach unterschiedlichem Programm, und insbesondere in einem jeweils angepassten zeitlichen Rhythmus für die Umschaltung betrieben werden, wobei die optimale Einstellung der Zeitpunkte für die Umschaltung der Belüftungsweiche von den Eigenschaften des Rottegutes abgeleitet werden können. Da diese Eigenschaften von der Kornverteilung, Feuchte, Schüttdichte, dem Strukturanteil und einer Reihe von nicht ohne weiteres quantifizierbaren Faktoren abhängen, wird der optimale zeitliche Rhythmus für die Umschaltung mit Vorteil empirisch ermittelt. Bei Verwendung von Rohluftströmen mit größeren Temperaturdifferenzen, wie beispielsweise Frischluft auf Feuchtkugeltemperatur und wasserdampfgesättigter Umluft, wird mit großen Enthalpieunterschieden gearbeitet und es kann die Steuerung der Belüftungsweiche in Abhängigkeit von der Änderung der Rotteguttemperaturen, welche bevorzugt im unteren Teil des Rottekörpers gemessen werden, erfolgen. Um das Kühlpotential der Prozessrohluft entsprechend zu maximieren, kann zumindest während der warmen Jahreshälfte die Wasserdampfsättigung der Frischluft beispielsweise in einem Füllkörperwäscher bei gleichzeitiger Abkühlung auf Feuchtkugeltemperatur erfolgen. Insgesamt wird mit dem erfindungsgemäßen Verfahren durch Sättigung der Frischluft oder die Abkühlung der Frischluft auf Feuchtkugeltemperatur vor Eintritt in den Rottekörper die gleiche Menge an Prozesswärme bei geringerem Verbrauch an Rottegutfeuchte aus dem Rottekörper ausgetragen werden. Eine Mischung gesättigter Frischluft und gesättigter Umluft kann zur Kondensatbildung führen, wobei diese Kondensatbildung erfahrungsgemäß und bevorzugt im Rottekörper selbst stattfindet, womit dem Rottekörper in bodennahen Schichten Feuchtigkeit zugeführt werden kann. In unmittelbarer Umgebung der Belüftungselemente, wie z.B. der Belüftungsdüsen oder Belüftungsrinnen wird das Rottegut durch Zufuhr von Frischluft vorübergehend rasch abgekühlt. Nach Umschalten der Belüftungsweiche kann in diesem Bereich aus der wärmeren und gesättigten Umluft Feuchte durch Kondensation an das Rottegut abgegeben werden. Durch entsprechende Steuerung der Belüftungsweiche bei gleichzeitiger Temperaturmessung im Rottekörper kann dieser Kondensationsmechanismus entsprechend gesteuert und optimiert werden.

Die von der Prozessabluft ausgetragene Rottegutfeuchte wird unter Berücksichtigung des im Prozess entstandenen Rottverlusts an organischer Trockensubstanz durch Nachbefeuchtung ergänzt, sodass ein prozesstechnisch günstiger Wassergehalt im Rottegut erhalten bleibt. Durch die erfindungsgemäße alternierende Belüftung wird der Nettowasserverlust des Rottekörpers zwar vermindert, ein großer Teil des Nachbefeuchtungsbedarfes muss aber weiterhin durch Besprühen der Oberfläche des Rottekörpers eingebracht werden.

In Anlagen mit mehreren Rottereaktoren kann der Einsatz der Umluft flexibler gestaltet werden, wenn diese aus einem für mehrere Reaktoren installierten Abluftsammler entnommen wird.

Die Erfindung wird nachfolgend anhand von in der Zeichnung schematisch dargestellten Ausführungsbeispielen näher erläutert. In dieser zeigen Fig. 1 eine schematische Darstellung von Vorrichtungen zum Belüften von Rotte, bei welchen die Luft von unten nach oben durch den Rottekörper gedrückt wird, nach dem Stand der Technik, Fig.2 eine erste Ausbildung der erfindungsgemäßen Ausgestaltung und Fig. 3 eine abgewandelte Ausbildung der erfindungsgemäßen Vorrichtung.

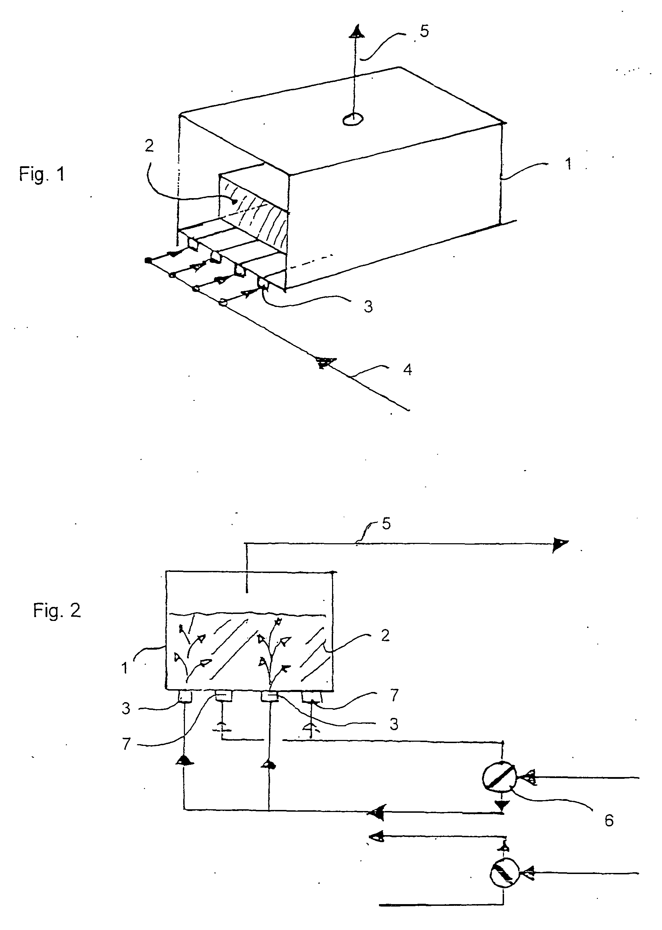

In Fig. 1 ist mit 1 ein geschlossener Rottereaktor bezeichnet, wobei in der Darstellung eine Seitenwand des geschlossenen Reaktors geöffnet ist. Über diese Öffnung kann ein Rottekörper 2 erkannt werden. Unterhalb des Rottekörpers 2 verlaufen Belüftungselemente 3, welche hier von Rinnen bzw. Düsenreihen gebildet sind und über Leitungen 4 mit Prozessrohluft gespeist werden. Die Prozessrohluft wird unter geringem Überdruck durch den Rottekörper 2 hindurchgepresst und aus dem geschlossenen Rottereaktor 1 über eine Leitung 5 als Prozessabluft abgezogen.

Bei der Darstellung nach Fig. 2 ist ersichtlich, dass in die Zuführung der Prozessrohluft eine Belüftungsweiche 6 eingeschaltet ist. Die Belüftungselemente sind in Gruppen unterteilt und neben einer Gruppe von Belüftungselementen 3 ist eine Gruppe von Belüftungselementen 7 vorgesehen, welche eine Teilgruppe der in Fig. 1 bereits dargestellten Belüftungselemente darstellt. Mittels der Belüftungsweiche kann nun zwischen den Reihen bzw. den Gruppen von Belüftungselementen 3 und 7 umgeschaltet werden, wodurch alternierend bzw. zyklisch Prozessrohluft eingetragen wird und stationären Strömungsverhältnissen im Rottekörper 2 entgegengewirkt wird.

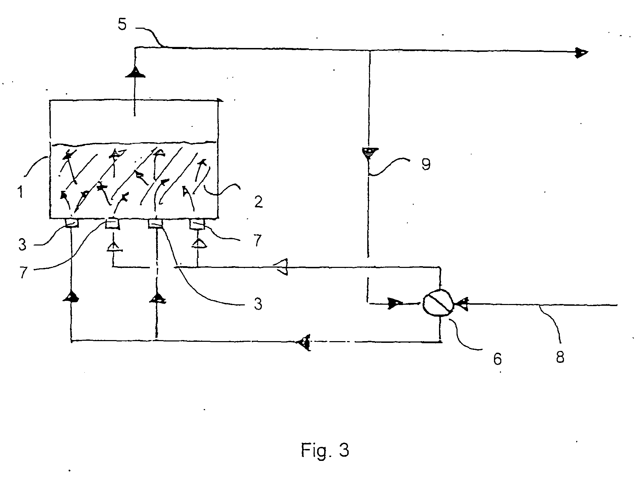

Bei der Darstellung nach Fig. 3 ist nun zusätzlich an die Belüftungsweiche 6 neben einer Zuleitung für die Prozessrohluft 8, welche bevorzugt von konditionierter Frischluft gebildet sein kann, eine Umluftleitung 9 angeschlossen, sodass nicht unterschiedliche Belüftungselemente alternierend oder zyklisch mit Prozessrohluft im wesentlichen gleicher Zusammensetzung, sondern auch mit voneinander verschiedener Zusammensetzung alternierend oder zyklisch beaufschlagt werden können. Der entsprechende nicht über die Umluftleitung geführte Anteil der Abluft gelangt als Nettoabluft zur Konditionierung bzw. nachträglichen Behandlung.

## Patentansprüche

1. Verfahren zum Belüften von Rotte bei welcher die Luft von unten nach oben durch den Rottekörper gedrückt wird, wobei die durch die Rotte drückend hindurchgeleitete Luft alternierend von Luft mit verschiedenen physikalischen und chemischen Eigenschaften und von an geometrisch voneinander verschiedenen Stellen eingeleiteter Luft gebildet wird, **dadurch gekennzeichnet, dass** Belüftungselemente in Gruppen unterteilt sind, die Gruppen von Belüftungselementen von Luft mit voneinander verschiedener Zusammensetzung beaufschlagt werden und die Beaufschlagung mittels einer Belüftungsweiche zwischen den Gruppen gewechselt wird.

2. Verfahren nach Anspruch 1, **dadurch gekennzeichnet, dass** als Rohluft Frischluft und/oder Umluft mit unterschiedlichem Wasserdampfgehalt, unterschiedlicher Temperatur und/oder unterschiedlicher Konditionierung alternierend durch parallel angeordnete Belüftungselemente geleitet wird.

3. Verfahren nach Anspruch 1 oder 2, **dadurch gekennzeichnet, dass** die alternierend beschickten Belüftungselemente von mit anderer Rohluft beschickten Belüftungselementen umgeben sind oder diese zwischen sich einschließen.

4. Verfahren nach Anspruch 1, 2 oder 3, **dadurch gekennzeichnet, dass** der Umschaltrhythmus der alternierend beschickten Belüftungselemente in Abhängigkeit von den im Rottekörper gemessenen Temperaturänderungen gesteuert wird.

## Claims

1. A method for aerating a rotting in which the air is pressed through the rotting body from bottom to top, wherein the air conducted through the rotting by pressing is alternately comprised of air having different physical and chemical properties and of air injected on geometrically different locations, **characterized in that** aeration elements are subdivided into groups, said groups of aeration elements are supplied with air of different compositions, and the supply is alternated between said groups by the aid of an aeration shunt.

2. A method according to claim 1, **characterized in that** fresh air and/or circulating air having different water vapour contents, different temperatures and/or different conditionings are alternately conducted as raw air through parallelly arranged aeration elements.

3. A method according to claim 1 or 2, **characterized in that** the alternately charged aeration elements are surrounded by, or enclose between them, aeration elements charged with a different raw air.

4. A method according to claim 1, 2 or 3, **characterized in that** the switching rhythm of the alternately charged aeration elements is controlled as a function of the temperature changes measured in the rotting body.

## Revendications

1. Procédé d'aération de compost, dans lequel l'air est forcé de bas en haut à travers le corps de compost, l'air acheminé de manière forcée à travers le compost étant constitué en alternance d'air de différentes propriétés physiques et chimiques et d'air introduit à des endroits géométriquement différents les uns des autres, **caractérisé en ce que** des éléments d'aération sont divisés en groupes, les groupes d'éléments d'aération sont alimentés en air de composition différente et l'alimentation est alternée entre les groupes au moyen d'un aiguillage d'aération.

2. Procédé selon la revendication 1, **caractérisé en ce que** l'air brut acheminé en alternance à travers des éléments d'aération disposés en parallèle est de l'air frais et/ou de l'air de circulation ayant une teneur en vapeur d'eau différente, une température différente et/ou un conditionnement différent.

3. Procédé selon la revendication 1 ou 2, **caractérisé en ce que** les éléments d'aération chargés en alternance sont entourés d'éléments d'aération chargés d'un air brut différent ou entourent respectivement ceux-ci.

4. Procédé selon la revendication 1, 2 ou 3, **caractérisé en ce que** le rythme de commutation des éléments d'aération chargés en alternance est commandé en fonction des variations de température mesurées dans le corps de compost.
